(19) Europäisches Patentamt
European Patent Office
Office européen des brevets

(11) EP 3 451 050 A1

(12) **EUROPEAN PATENT APPLICATION**

(43) Date of publication:
06.03.2019 Bulletin 2019/10

(51) Int Cl.:
*G02C 7/04* (2006.01)      *A61F 2/16* (2006.01)

(21) Application number: 18191430.0

(22) Date of filing: 29.08.2018

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 30.08.2017 US 201715690667

(71) Applicant: Johnson & Johnson Vision Care, Inc.
Jacksonville, FL 32256 (US)

(72) Inventors:
• WOOLEY, C. Benjamin
Jacksonville, FL Florida 32256 (US)
• CHEN, Minghan
Jacksonville, FL Florida 32256 (US)

(74) Representative: Carpmaels & Ransford LLP
One Southampton Row
London WC1B 5HA (GB)

(54) **ATORIC SURFACES TO MINIMIZE SECONDARY ASTIGMATISM IN CONTACT LENSES FOR THE CORRECTION OF ASTIGMATISM**

(57) The present disclosure relates to ophthalmic devices such as ophthalmic lenses. An ophthalmic device may comprise an aspherical first surface and an atoric second surface, disposed opposite the first surface and configured to be disposed adjacent an eye of a user. The second surface may comprise a first conic constant along a first meridian and a second conic constant along a second meridian. The ophthalmic lens may be configured to minimize spherical aberration along the first meridian and the second meridian.

# FIG. 10B

EP 3 451 050 A1

**Description**

BACKGROUND OF THE DISCLOSURE

1. Field of the Disclosure

The present disclosure relates to ophthalmic devices, such as wearable lenses, including contact lenses, implantable lenses, including inlays and onlays and any other type of device comprising optical components, and more particularly, to ophthalmic devices having an atoric surface for the correction of astigmatism.

2. Discussion of the Related Art

Ophthalmic devices, such as contact lenses, currently are utilized to correct vision defects such as myopia (nearsightedness), hyperopia (farsightedness), presbyopia and astigmatism. However, properly designed lenses may be utilized to enhance vision as well as to correct vision defects.

Astigmatism is a type of refractive error in which the eye does not focus light symmetrically on the retina. Astigmatism significantly degrades the patient's retinal image quality and therefore their perceived quality of vision. In addition to an asymmetric blurring of the image, higher degrees of astigmatism may cause symptoms such as squinting, eye strain, fatigue, or even headaches. Astigmatism in the eye can originate from asymmetries about the optical axis of both the cornea and the crystalline lens.

A contact lens with cylindrical power may be employed to address astigmatism. Current contact lens designs correct the primary astigmatism, but induce secondary astigmatism that varies with the value of the spherical refraction. Secondary astigmatism, analogous to spherical aberration (SPHA), can impact the quality and consistency of vision even when the refractive error and the primary astigmatism are corrected. When the secondary astigmatism varies with spherical refraction, the product offering does not provide a consistent visual experience to all patients.

SUMMARY OF THE DISCLOSURE

The present disclosure relates to ophthalmic devices that comprise atoric surfaces, which may be configured for the correction of astigmatism. In certain aspects, the present disclosure provides for contact lenses and a method for designing contact lenses that have zero secondary astigmatism and therefore provide improved and more consistent vision performance. Additionally, or alternatively, the spherical aberration of the contact lens can be specified to provide consistent vision.

The present disclosure relates to ophthalmic devices such as ophthalmic lenses. An ophthalmic device may comprise an aspherical first surface and an atoric second surface, disposed opposite the first surface and configured to be disposed adjacent an eye of a user. The second surface may comprise a first conic constant along a first meridian and a second conic constant along a second meridian. The ophthalmic lens may be configured to minimize spherical aberration along the first meridian and the second meridian.

**BRIEF DESCRIPTION OF THE DRAWINGS**

The foregoing and other features and advantages of the disclosure will be apparent from the following, more particular description of preferred embodiments of the disclosure, as illustrated in the accompanying drawings.

Figure 1 illustrates an exemplary representation of toric surface showing sphere meridian and cylinder meridian each a slice through a unique sphere.

Figure 2 illustrates an exemplary representation of a cross section of a spherical and aspheric surface.

Figure 3 illustrates a schematic representation of an atoric surface.

Figure 4 illustrates a power profile of an example lens along the meridian direction with a smooth cornea curvature.

Figure 5 illustrates a power profile of an example lens with double aspherical curvature, the power profile taken along the meridian direction with steep cornea curvature.

Figure 6 illustrates a power profile of an example lens is with conventional sphere curvature.

Figure 7A illustrates a plot of the spherical aberration along a cylindrical meridian with respect to spherical optical power.

Figure 7B illustrates a plot of the spherical aberration along a non-cylindrical meridian with respect to spherical optical power.

Figure 8 illustrates an example plot indicated the difference between primary astigmatism (SA1) and secondary astigmatism (SA2) with respect to spherical optical power. Different curves (shown with different line styles) illustrate the value with different cylindrical power. Areas covered by the dashed rectangular boxes indicate that at the extreme region (e.g., -9 to -12D, -2.75D cyl) the secondary astigmatism values rather high.

Figures 9A and 9B illustrates two embodiments of the optical designs, where Figure 9A shows a front surface of a lens comprises an atoric structured surface and a back surface of the lens comprising a regular sphere/aspherical structure, and where Figure 9B shows a back surface of a lens comprising an atoric structure and a front surface of the lens comprising a spherical/aspherical surface.

Figures 10A and 10B illustrates a plot of spherical aberration of cylindrical lenses with different spherical power (-9D to +6D) and different cylindrical power (-0.75D to - 3.25D) for lenses with regular spherical surface Figure 10A and atoric surface Figure 10B respectively. The unit of spherical aberration is in $D/mm^2$. For lens with regular spherical surface, the spherical aberration ranges from around -0.07 to -0.01 $D/mm^2$. While for atoric structure, the spherical aberration covers less range (from around -0.01 to 0.01 $D/mm^2$).

Figures 11A and 11B illustrates a power profile of Figure 11A a manufactured regular spherical surface lens and Figure 11B a lens with atroic structure (atoric back surface and aspherical front surface), respectively. The solid curve shows the power profile along non-astigmatism meridian direction and the dashed curve indicates the power profile along astigmatism direction respectively.

## DETAILED DESCRIPTION

Ophthalmic devices may include implantable device and/or wearable devices, such as contact lenses. Conventional contact lenses comprise polymeric structures with specific shapes to correct various vision problems.

As part of a typical eye examination the Eye Care Professional (ECP) may determine the contact lens prescription required to correct the patients' refractive error. This prescription may specify the refractive power, cylindrical power, and/or cylinder axis of the contact lens, which may be used in determining the design or selection of a design of a contact lens.

Typical contact lens designs for the correction of astigmatism may comprise an optic zone that is a section of a spherical surface on the front of the lens and a section of a toric surface on the back of the lens. The spherical refraction may be determined by the radius of the front surface within the optic zone considering the lens thickness, the index of refraction, and/or the radius of the back surface along the sphere axis.

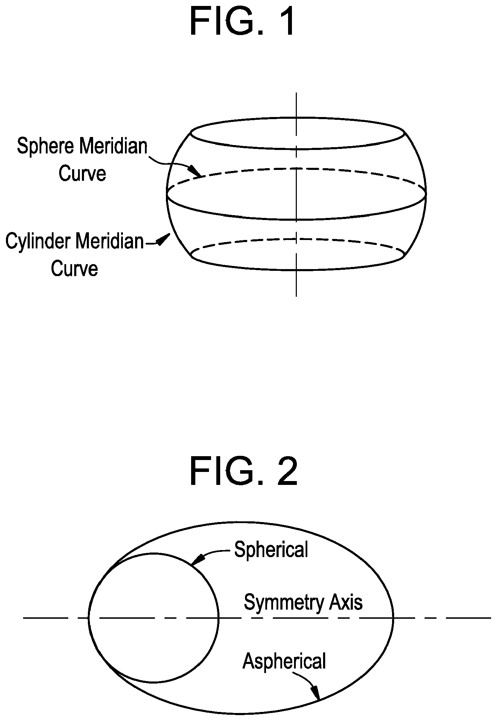

FIG. 1 illustrates an example toric surface, which has a unique curvature or radius along the two orthogonal meridians (e.g. the sphere meridian and the cylinder meridian). The two unique curvatures may provide two unique powers along the two orthogonal axes. This difference in curvature may be used to provide astigmatism correction and the orientation of the sphere and cylinder meridians may be specified by the required cylinder axis. Maintaining the proper axis for the lens on-eye requires orientation stabilization, which can be provided by many different methods.

However, SPHA (e.g., primary and/or secondary astigmatism) may be introduced due to the refraction nature of the front spherical surface and the fact that the back surface is spherical along the two orthogonal meridians. SPHA can be corrected or controlled to provide a constant value (e.g., near constant) across different refractive powers by making the front surface within the optic zone aspheric. As described herein, SPHA may be corrected or controlled to provide comparatively less incremental change in power from a center to an edge of an atoric lens, when compared to a spherical lens.

FIG. 2 shows a cross section of a spherical and aspheric surface showing the difference. Further, because the toric back surface has different radii along the two orthogonal meridians, the amount of SPHA along two different meridian directions is different whether or not the front surface is spherical or aspheric. This difference in SPHA along the sphere and cylinder meridians is, in a Zernike aberration sense, what is referred to as secondary astigmatism. As described herein, this secondary astigmatism can be corrected by making the back surface within the optic zone atoric. Alternatively, a front surface of a lens may comprise an atoric surface, as described herein.

### Optical lens design, visual simulation and its application:

The present disclosure relates to a lens design comprising an aspherical lens with aspherical first (e.g., front) surface and an atoric second (e.g., back) surface with different conic constants along the two orthogonal (sphere and cylinder) meridians to minimize the secondary astigmatism. As such, the lens design may be configured to correct the SPHA and secondary SPHA simultaneously in a contact lens intended for the correction of both refractive error and astigmatic error. Although reference is made to a lens having an atoric back surface that is disposed on or adjacent an eye of a wearer, alternatively a front surface of the lens may be atoric and may realize the same advantages as described herein over a spherical lens. As an example, an ophthalmic device in accordance with aspects of the present disclosure may be configured to correct ocular astigmatism along one or more meridians based upon the optical and surface configuration of the device, as detailed herein.

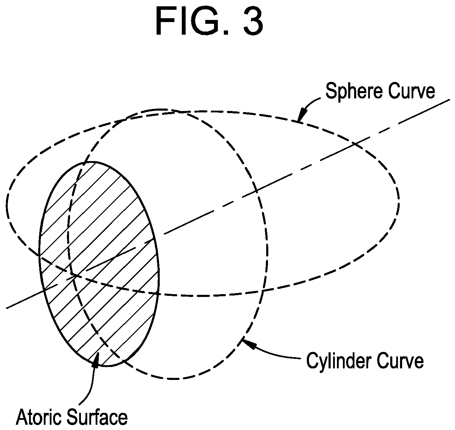

FIG. 3 shows a schematic representation of an atoric surface. As an illustrative example, a series of lens designs were made with spherical power covering from -9D to +6D and the cylindrical power of -0.75D, -1.25D, -1.75D, -2.25D, -2.75D, -3.25D. For the prepared lenses, the back surfaces shared the same aspherical curvature (r=7.85 and k=-0.26) along the sphere axis. (The back surface along the sphere axis could also be spherical with k=0, and could have a different r value.) For each individual spherical power, an aspherical front surface is designed in order to have overall zero SPHA along the sphere meridian direction. A different aspherical curvature may be designed with each individual cylindrical power (cylinder refraction) to minimize the SPHA along the cylinder axis. However, as described herein below, one or more surfaces of the lens may be configured to correct an ocular astigmatism by exhibiting a non-zero SPHA along one or more of the sphere meridian and the cylindrical meridian.

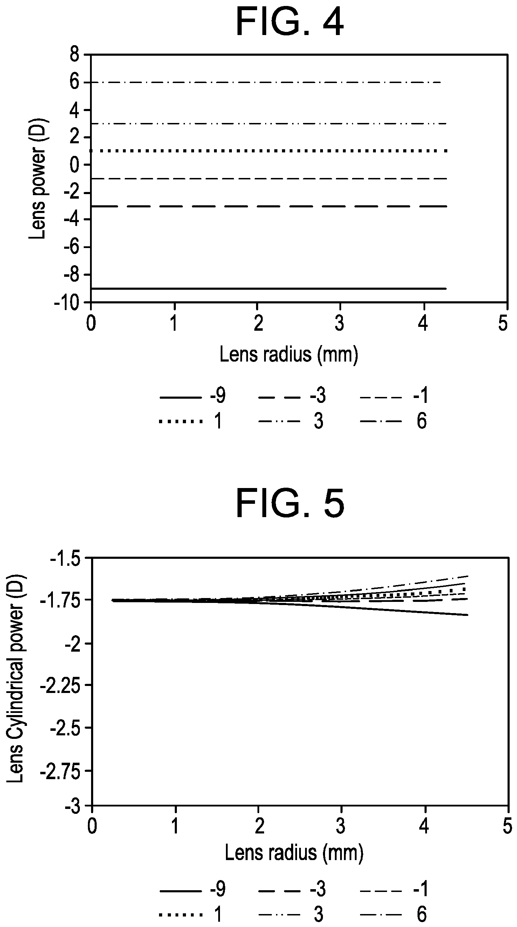

As shown in FIG. 4, zero SPHA (or about zero SPHA, within manufacturing tolerance) was obtained along the sphere meridian direction. As an illustrative example, the generally flat power profile along the radius of the sphere meridian illustrated in FIG. 5 demonstrates that the subject lens of FIGS. 4-5 is configured to minimize SPHA along the sphere meridian.

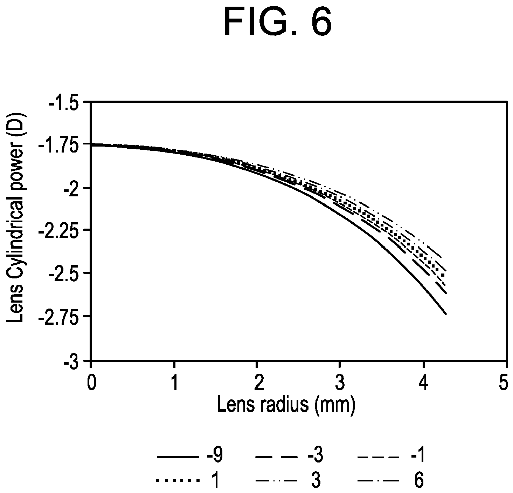

FIG. 5 indicates the power profile (for a lens with double aspherical curvatures) along the astigmatism direction, a very small amount of power variation (-0.006D/ mm2) was observed, which indicates the SPHA was minimized along that direction. For comparison, FIG. 6 shows the power profile of a lens with a conventional toric back curvatures. The comparatively large amount of power variation over the plot in FIG. 5 indicates the large amount of SPHA (~0.06D/mm2) along the direction. As such, minimizing SPHA may comprise providing a lens with less power variation from a center to edge of the lens as compared to a conventional lens having a toric back surface. Minimizing SPHA may comprise providing a lens with less power variation from a center to edge of the lens as compared to a conventional lens having at least one spherical surface.

**Examples:**

As an example, a clinical data set referenced herein as the salmon data represents a data set taken across 10 sites, as described in "Normal-eye Zernike coefficients and root-mean-square wavefront errors," J Cataract Refract Surg. 2006 Dec; 32(12):2064-74. The salmon data includes patient information such as gender, age, OD/OS, and other variables. The data represents measurements comprising spherical refraction, cylinder refraction, cylinder axis, and Zernike (pupil).

In accordance with aspects of the present disclosure, and based upon analysis of the salmon data, wavefront data was fitted along two meridian directions, resulting in the following equations for the two meridians:

$$\theta_1 = \mathrm{atan}(Z(2,2)/Z(2,-2))/2$$

$$\theta_2 = \theta_1 + pi/2$$

Along each meridian direction, a quadratic equation was employed for fitting the data:

$$SA(\theta_1) = c1(\theta_1) + c2(\theta_1) * P(\theta_1) + c3(\theta_1) * P(\theta_1)^2$$

$$SA(\theta_2) = c1(\theta_2) + c2(\theta_2) * P(\theta_2) + c3(\theta_2) * P(\theta_2)^2$$

$$2nd\ astigmatism = SA(\theta_1) - SA(\theta_2)$$

As such, to correct secondary astigmatism, a lens design may be configured to meet the following relationships:

Lens SA(01)=0; SA(02)=0; and/or
Lens 2nd astg=SA(01) - SA(θ2)=0.

By considering the relationships of the spherical aberration described herein, ophthalmic lenses may be configured to correct the SPHA and secondary SPHA simultaneously in a contact lens intended for the correction of both refractive error and astigmatic error. Correct of the SPHA and secondary SPHA may be realized by providing a lens that exhibits a power variation along a meridian that is below a threshold (e.g., .05D/mm^2., .01 D/mm^2, .005D/mm^2, .001D/mm^2). Correct of the SPHA and secondary SPHA may be realized by providing a lens that exhibits a power variation from center to edge of the lens that is comparatively less than the power variation exhibited by a substantially similar lens having the same radius but comprising a spherical surface.

In certain aspects, ocular astigmatism for a particular wearer or group of wearers may be such that a corrective lens may be targeted or customized to provide a target amount of SPHA (instead of configuring the lens for zero secondary astigmatism). As an example, various populations may be studied and the ocular characteristics of the wearers of the population may be aggregated (and/or further processed (e.g., averaged)).

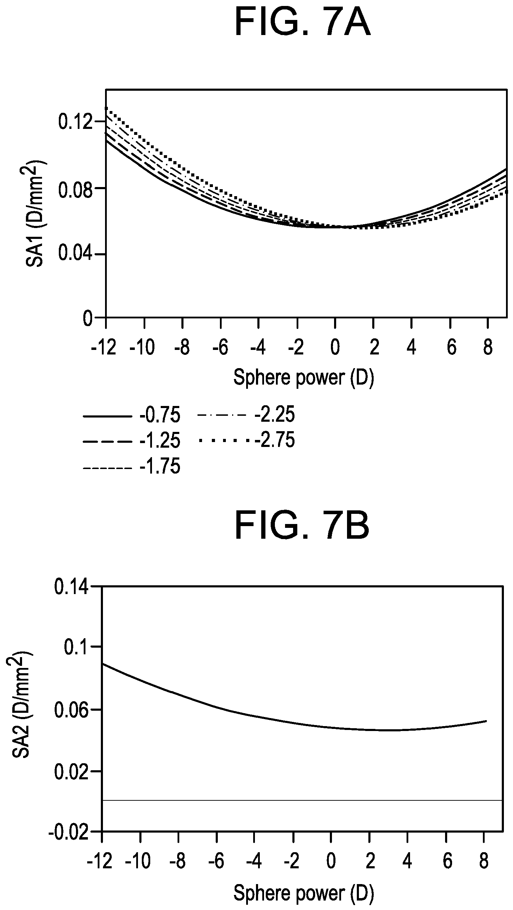

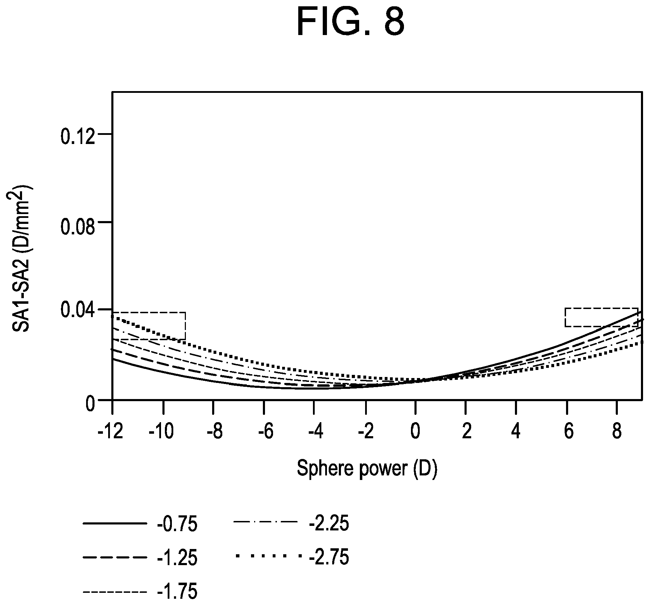

Figures 7A and 7B indicate the ocular spherical aberration along cylindrical meridian and non-cylindrical direction respectively. Figure 8 indicates the difference between ocular primary astigmatism and ocular secondary astigmatism. Different curves (shown with different line styles) illustrate the value with different cylindrical power. Areas covered by the dashed rectangular boxes indicated that at the extreme regions (e.g., -9 to -12D, -2.75D cyl) the secondary astigmatism values are higher than the regions closer to 0D. As such, a device such as a lens which has an opposite secondary astigmatism value can be used to compensate ocular secondary astigmatism and offer better visual performance. In particular, devices such as lenses may be configured to specifically correct for ocular astigmatism by compensating the ocular spherical aberration exhibited along one or more meridians. As an example, a device may be configured to exhibit a select spherical aberration along a spherical and/or cylindrical meridian (e.g., mathematically orthogonal meridians) to be customized for correction of an individual or group of individuals exhibiting the ocular astigmatism represented in FIGS. 7-8.

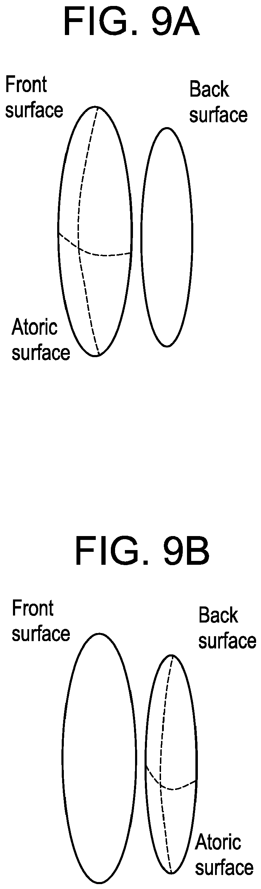

Figures 9A and 9B illustrate two embodiments of the optical designs, where Figure 9A shows a front surface of a lens comprises an atoric structured surface and a back surface of the lens comprising a regular sphere/aspherical structure, and where Figure 9B shows a back surface of a lens comprising an atoric structure and a front surface of the lens comprising a spherical/aspherical surface. As described herein, a lens having at least one surface comprising the atoric structured surface may exhibit a correction of primary SPHA and secondary SPHA.

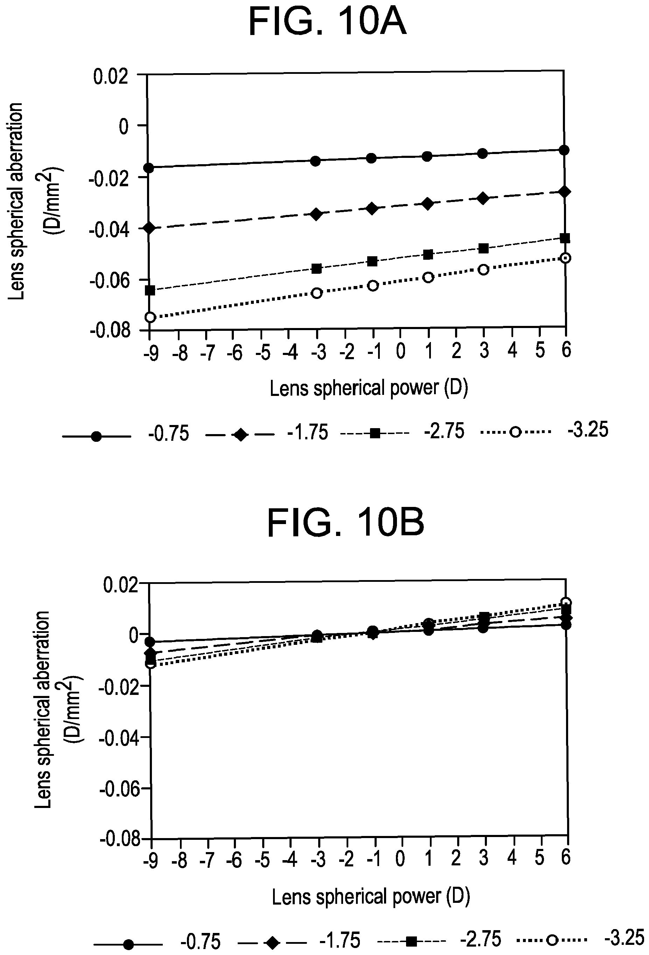

Figures 10A and 10B illustrate a plot of spherical aberration of cylindrical lenses with different spherical power (-9D to +6D) and different cylindrical power (-0.75D to - 3.25D) for lenses with (a) regular spherical surface and (b) atoric surface, respectively. The unit of spherical aberration is in $D/mm^2$. For lens with regular spherical surface, the spherical aberration ranges from around -0.07 to -0.01 $D/mm^2$. However, for atoric structure, the spherical aberration covers less range (from around -0.01 to 0.01 $D/mm^2$).

Therefore, it is observed that the atoric structure in a lens exhibits an improved secondary SPHA relative to the lens with the spherical surface. Thus, the inclusion of the atoric structure may minimize the SPHA.

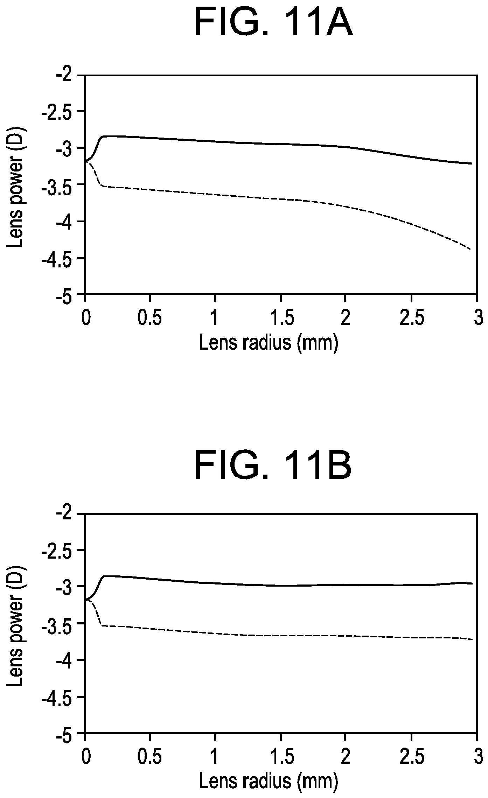

Figures 11A and 11B illustrate a power profile of (a) a manufactured regular spherical surface lens and (b) a lens with atroic structure (atoric back surface and aspherical front surface), respectively. The solid curve shows the power profile along non-astigmatism meridian direction and the dashed curve indicates the power profile along astigmatism direction respectively. The difference between the two curves indicates the astigmatism power. In Fig 11A, there is a significant increment of the cylindrical power from lens center to its edge, which indicates the significant amount of secondary astigmatism. While in Fig 11B, the increment of cylindrical power at lens edge is not that significant indicating almost zero (or zero) secondary astigmatism.

**Aspects:**

In various aspects, the present disclosure may pertain to one or more of the following aspects.

Aspect 1: An ophthalmic lens comprising: a first surface; and an atoric second surface, disposed opposite the first surface, wherein the second surface comprises a first conic constant along a first meridian and a second conic constant along a second meridian, wherein the first meridian is orthogonal to the second meridian, and wherein the ophthalmic lens is configured to minimize spherical aberration along the first meridian and the second meridian.

Aspect 2: The ophthalmic lens according to aspect 1, wherein the ophthalmic lens comprises a contact lens, and wherein the first surface is a front surface and the second surface is a rear surface configured to be disposed adjacent an eye of a wearer.

Aspect 3: The ophthalmic lens according to aspect 1, wherein the ophthalmic lens comprises a contact lens, and wherein the first surface is a rear surface configured to be disposed adjacent an eye of a wearer and the second surface is a front surface.

Aspect 4: The ophthalmic lens according to any one of aspects 1-3, wherein the first surface comprises one of an aspherical surface, a spherical surface, or a diffractive surface.

Aspect 5: The ophthalmic lens according to any one of aspects 1-4, wherein the ophthalmic lens has a spherical power from -9D to +6D.

Aspect 6: The ophthalmic lens according to any one of aspects 1-5, wherein the ophthalmic lens has a cylindrical power from -0.75D to -3.25D.

Aspect 7: The ophthalmic lens according to any one of aspects 1-6, wherein the first meridian is a sphere meridian and the second meridian is a cylinder meridian.

Aspect 8: The ophthalmic lens according to any one of aspects 1-7, wherein the ophthalmic lens exhibits about zero spherical aberration along the first meridian.

Aspect 9: The ophthalmic lens according to any one of aspects 1-8, wherein the ophthalmic lens exhibits zero spherical aberration along the first meridian.

Aspect 10: The ophthalmic lens according to any one of aspects 1-9, wherein the ophthalmic lens exhibits less than .001D/mm^2 spherical aberration along the second meridian.

Aspect 11: The ophthalmic lens according to any one of aspects 1-9, wherein the ophthalmic lens exhibits less than .05D/mm^2 spherical aberration along the second meridian when configured to exhibit a cylindrical optical power of -3.25D.

Aspect 12: The ophthalmic lens according to any one of aspects 1-9, wherein the ophthalmic lens exhibits less than .04D/mm^2 spherical aberration along the second meridian when configured to exhibit a cylindrical optical power of between -2.75D and - 3.25D.

Aspect 13: The ophthalmic lens according to any one of aspects 1-9, wherein the ophthalmic lens exhibits less than .002D/mm^2 spherical aberration along the second meridian when configured to exhibit a cylindrical optical power of between -1.75D and - 3.25D.

Aspect 14: The ophthalmic lens according to any one of aspects 1-9, wherein the ophthalmic lens exhibits less than .001D/mm^2 spherical aberration along the second meridian when configured to exhibit a cylindrical optical power of between -0.75D and - 3.25D.

Aspect 15: An ophthalmic lens comprising: an aspherical front surface; and an atoric rear surface, disposed opposite the front surface, wherein the rear surface comprises a first conic constant along a sphere meridian and a second conic constant along a cylinder meridian, wherein the sphere meridian is orthogonal to the cylinder meridian, and wherein the ophthalmic lens is configured to minimize spherical aberration of the lens along the sphere meridian and the cylinder meridian.

Aspect 16: The ophthalmic lens according to aspect 15, wherein the ophthalmic lens comprises a contact lens.

Aspect 17: The ophthalmic lens according to aspect 16, wherein the contact lens comprises a soft or hybrid contact lens.

Aspect 18: The ophthalmic lens according to any one of aspects 15-17, wherein the ophthalmic lens has a spherical power from -9D to +6D.

Aspect 19: The ophthalmic lens according to any one of aspects 15-18, wherein the ophthalmic lens has a cylindrical power from -0.75D to -3.25D.

Aspect 20: The ophthalmic lens according to any one of aspects 15-19, wherein the ophthalmic lens exhibits about zero spherical aberration along the sphere meridian.

Aspect 21: The ophthalmic lens according to any one of aspects 15-20, wherein the ophthalmic lens exhibits zero spherical aberration along the sphere meridian.

Aspect 22: The ophthalmic lens according to any one of aspects 15-21, wherein the ophthalmic lens exhibits less than .001 D/mm^2 spherical aberration along the cylinder meridian.

Aspect 23: The ophthalmic lens according to any one of aspects 15-21, wherein the ophthalmic lens exhibits less than .05D/mm^2 spherical aberration along the cylinder meridian when configured to exhibit a cylindrical optical power of -3.25D.

Aspect 24: The ophthalmic lens according to any one of aspects 15-21, wherein the ophthalmic lens exhibits less than .04D/mm^2 spherical aberration along the cylinder meridian when configured to exhibit a cylindrical optical power of between - 2.75D and -3.25D.

Aspect 25: The ophthalmic lens according to any one of aspects 15-21, wherein the ophthalmic lens exhibits less than .002D/mm^2 spherical aberration along the cylinder meridian when configured to exhibit a cylindrical optical power of between - 1.75D and -3.25D.

Aspect 26: The ophthalmic lens according to any one of aspects 15-21, wherein the ophthalmic lens exhibits less than .001D/mm^2 spherical aberration along the cylinder meridian when configured to exhibit a cylindrical optical power of between - 0.75D and -3.25D.

Aspect 27: An ophthalmic lens comprising: an aspherical first surface; and an atoric second surface, disposed opposite the first surface; wherein the second surface comprises a first conic constant along a first meridian and a second conic constant along a second meridian, wherein the first meridian is orthogonal to the second meridian, and wherein the ophthalmic lens exhibits less spherical aberration along the second merdian compared to a comparative lens consisting essentially of the same configuration as the ophthalmic lens but without the atoric second surface.

Aspect 28: The ophthalmic lens according to aspect 27, wherein the first surface is a front surface and the second surface is a rear surface configured to be disposed adjacent an eye of a wearer.

Aspect 29: The ophthalmic lens according to aspect 27, wherein the first surface is a rear surface configured to be disposed adjacent an eye of a wearer and the second surface is a front surface.

Aspect 30: The ophthalmic lens according to any one of aspects 27-29, wherein the ophthalmic lens has a spherical power from -9D to +6D.

Aspect 31: The ophthalmic lens according to any one of aspects 27-30, wherein the ophthalmic lens has a cylindrical power from -0.75D to -3.25D.

Aspect 32: The ophthalmic lens according to any one of aspects 27-31, wherein the first meridian is a sphere meridian and the second meridian is a cylinder meridian.

Aspect 33: The ophthalmic lens according to any one of aspects 27-32, wherein the ophthalmic lens exhibits about zero spherical aberration along the first meridian.

Aspect 34: The ophthalmic lens according to any one of aspects 27-33, wherein the ophthalmic lens exhibits zero spherical aberration along the first meridian.

Aspect 35: The ophthalmic lens according to any one of aspects 27-34, wherein the ophthalmic lens exhibits less than .001 D/mm^2 spherical aberration along the second meridian.

Aspect 36: The ophthalmic lens according to any one of aspects 27-34, wherein the ophthalmic lens exhibits less than .05D/mm^2 spherical aberration along the second meridian when configured to exhibit a cylindrical optical power of -3.25D.

Aspect 37: The ophthalmic lens according to any one of aspects 27-34, wherein the ophthalmic lens exhibits less than .04D/mm^2 spherical aberration along the second meridian when configured to exhibit a cylindrical optical power of between -2.75D and - 3.25D.

Aspect 38: The ophthalmic lens according to any one of aspects 27-34, wherein the ophthalmic lens exhibits less than .002D/mm^2 spherical aberration along the second meridian when configured to exhibit a cylindrical optical power of between - 1.75D and -3.25D.

Aspect 39: The ophthalmic lens according to any one of aspects 27-34, wherein the ophthalmic lens exhibits less than .001 D/mm^2 spherical aberration along the second meridian when configured to exhibit a cylindrical optical power of between - 0.75D and -3.25D.

Aspect 40: The ophthalmic lens according to any one of aspects 27-39, wherein the ophthalmic lens exhibits less power variation measured from a lens center to a lens edge as compared to the comparative lens.

Aspect 41: The ophthalmic lens according to any one of aspects 27-39, wherein the ophthalmic lens exhibits less power variation measured between a lens center to a position within an outer half of the lens radius, as compared to the comparative lens.

Aspect 42: The ophthalmic lens according to any one of aspects 27-41, wherein the comparative lens comprises an aspherical first surface and a non-atoric second surface.

Aspect 43: The ophthalmic lens according to any one of aspects 27-41, wherein the comparative lens comprises an aspherical first surface and a toric second surface.

Aspect 44: The ophthalmic lens according to any one of aspects 27-41, wherein the comparative lens comprises an aspherical first surface and an aspherical second surface.

Aspect 45: The ophthalmic lens according to any one of aspects 27-41, wherein the comparative lens comprises an aspherical first surface and a spherical second surface.

Aspect 46: An ophthalmic lens comprising: a first surface; and an atoric second surface, disposed opposite the first surface, wherein the second surface comprises a first conic constant along a first meridian and a second conic constant along a second meridian, wherein the first meridian is orthogonal to the second meridian, wherein the ophthalmic lens is configured to exhibit a zero spherical aberration along the first meridian and a target spherical aberration along the second meridian, and wherein the target spherical aberration along the second meridian is configured based on correction of a target ocular secondary astigmatism level.

Aspect 47: The ophthalmic lens according to aspect 46, wherein the ophthalmic lens comprises a contact lens, and wherein the first surface is a front surface and the second surface is a rear surface configured to be disposed adjacent an eye of a wearer.

Aspect 48: The ophthalmic lens according to aspect 46, wherein the ophthalmic lens comprises a contact lens, and wherein the first surface is a rear surface configured to be disposed adjacent an eye of a wearer and the second surface is a front surface.

Aspect 49: The ophthalmic lens according to any one of aspects 46-48, wherein the first surface comprises one of an aspherical surface, a spherical surface, or a diffractive surface.

Aspect 50: The ophthalmic lens according to any one of aspects 46-49, wherein the ophthalmic lens has a spherical power from -9D to +6D.

Aspect 51: The ophthalmic lens according to any one of aspects 46-50, wherein the ophthalmic lens has a cylindrical power from -0.75D to -3.25D.

Aspect 52: The ophthalmic lens according to any one of aspects 46-51, wherein the first meridian is a sphere meridian and the second meridian is a cylinder meridian.

Aspect 53: The ophthalmic lens according to any one of aspects 46-52, wherein the ophthalmic lens exhibits about zero spherical aberration along the first meridian.

Aspect 54: The ophthalmic lens according to any one of aspects 46-53, wherein the ophthalmic lens exhibits zero spherical aberration along the first meridian.

Aspect 55: The ophthalmic lens according to any one of aspects 46-54, wherein the target spherical aberration along the second meridian is less than .001 D/mm^2.

Aspect 56: The ophthalmic lens according to any one of aspects 46-54, wherein the target spherical aberration along the second meridian is less than .05D/mm^2 when the ophthalmic lens is configured to exhibit a cylindrical optical power of -3.25D.

Aspect 57: The ophthalmic lens according to any one of aspects 46-54, wherein the target spherical aberration along the second meridian is less than.04D/mm^2 when the ophthalmic lens is configured to exhibit a cylindrical optical power of between -2.75D and -3.25D.

Aspect 58: The ophthalmic lens according to any one of aspects 46-54, wherein the target spherical aberration along the second meridian is less than .002D/mm^2 when the ophthalmic lens is configured to exhibit a cylindrical optical power of between - 1.75D and -3.25D.

Aspect 59: The ophthalmic lens according to any one of aspects 46-54, wherein the target spherical aberration along the second meridian is less than .001D/mm^2 spherical aberration along the second meridian when the ophthalmic lens is configured to exhibit a cylindrical optical power of between -0.75D and -3.25D.

Aspect 60: The ophthalmic lens according to any one of aspects 46-59, wherein the target spherical aberration along the second meridian is based on an inverse of the target ocular secondary astigmatism level.

Aspect 61: The ophthalmic lens according to any one of aspects 46-59, wherein the target spherical aberration along the second meridian is an inverse of the target ocular secondary astigmatism level.

Aspect 62: An ophthalmic lens comprising: a first surface; and an atoric second surface, disposed opposite the first surface, wherein the second surface comprises a first conic constant along a first meridian and a second conic constant along a second meridian, wherein the first meridian is orthogonal to the second meridian, wherein the ophthalmic lens is configured to exhibit a first target spherical aberration along the first meridian and a second target spherical aberration along the second meridian, wherein the first target spherical aberration along the first meridian is configured based on correction of a target ocular primary astigmatism level, and wherein the second target spherical aberration along the second meridian is configured based on correction of a target ocular secondary astigmatism level.

Aspect 63: The ophthalmic lens according to aspect 62, wherein the ophthalmic lens comprises a contact lens, and wherein the first surface is a front surface and the second surface is a rear surface configured to be disposed adjacent an eye of a wearer.

Aspect 64: The ophthalmic lens according to aspect 62, wherein the ophthalmic lens comprises a contact lens, and wherein the first surface is a rear surface configured to be disposed adjacent an eye of a wearer and the second surface is a front surface.

Aspect 65: The ophthalmic lens according to any one of aspects 62-64, wherein the first surface comprises one of an aspherical surface, a spherical surface, or a diffractive surface.

Aspect 66: The ophthalmic lens according to any one of aspects 62-65, wherein the ophthalmic lens has a spherical power from -9D to +6D.

Aspect 67: The ophthalmic lens according to any one of aspects 62-66, wherein the ophthalmic lens has a cylindrical power from -0.75D to -3.25D.

Aspect 68: The ophthalmic lens according to any one of aspects 62-67, wherein the first meridian is a sphere meridian and the second meridian is a cylinder meridian.

Aspect 69: The ophthalmic lens according to any one of aspects 62-68, wherein the first target spherical aberration is about zero.

Aspect 70: The ophthalmic lens according to any one of aspects 62-69, wherein the first target spherical aberration is zero.

Aspect 71: The ophthalmic lens according to any one of aspects 62-70, wherein the first target spherical aberration is based on an inverse of the target ocular primary astigmatism level.

Aspect 72: The ophthalmic lens according to any one of aspects 62-70, wherein the first target spherical aberration is an inverse of the target ocular primary astigmatism level.

Aspect 73: The ophthalmic lens according to any one of aspects 62-72, wherein the second target spherical aberration along the second meridian is less than .001D/mm^2.

Aspect 74: The ophthalmic lens according to any one of aspects 62-72, wherein the second target spherical aberration along the second meridian is less than .05D/mm^2 when the ophthalmic lens is configured to exhibit a cylindrical optical power of -3.25D.

Aspect 75: The ophthalmic lens according to any one of aspects 62-72, wherein the second target spherical aberration along the second meridian is less than .04D/mm^2 when the ophthalmic lens is configured to exhibit a cylindrical optical power of between -2.75D and -3.25D.

Aspect 76: The ophthalmic lens according to any one of aspects 62-72, wherein the second target spherical aberration along the second meridian is less than .002D/mm^2 when the ophthalmic lens is configured to exhibit a cylindrical optical power of between -1.75D and -3.25D.

Aspect 77: The ophthalmic lens according to any one of aspects 62-72, wherein the second target spherical aberration along the second meridian is less than .001D/mm^2 spherical aberration along the second meridian when the ophthalmic lens is configured to exhibit a cylindrical optical power of between -0.75D and -3.25D.

Aspect 78: The ophthalmic lens according to any one of aspects 62-72, wherein the second target spherical aberration along the second meridian is based on an inverse of the target ocular secondary astigmatism level.

Aspect 79: The ophthalmic lens according to any one of aspects 62-72, wherein the second target spherical aberration along the second meridian is an inverse of the target ocular secondary astigmatism level.

Although shown and described in what is believed to be the most practical and preferred embodiments, it is apparent that departures from specific designs and methods described and shown will suggest themselves to those skilled in the art and may be used without departing from the spirit and scope of the disclosure. The present disclosure is not restricted to the particular constructions described and illustrated, but should be constructed to cohere with all modifications that may fall within the scope of the appended claims. Moreover, the recitation of the term comprising may include consisting essentially of and/or consisting of such that support is found herein for such terms by the use of the term comprising.

## Claims

1. An ophthalmic lens comprising:

   a first surface; and
   an atoric second surface, disposed opposite the first surface,
   wherein the second surface comprises a first conic constant along a first meridian and a second conic constant along a second meridian,
   wherein the first meridian is orthogonal to the second meridian, and
   wherein the ophthalmic lens is configured to minimize spherical aberration along the first meridian and the second meridian.

2. The ophthalmic lens according to claim 1, wherein the ophthalmic lens comprises a contact lens, and wherein the first surface is a front surface and the second surface is a rear surface configured to be disposed adjacent an eye of a wearer.

3. The ophthalmic lens according to claim 1, wherein the ophthalmic lens comprises a contact lens, and wherein the first surface is a rear surface configured to be disposed adjacent an eye of a wearer and the second surface is a front surface.

4. The ophthalmic lens according to any of claims 1 to 3, wherein the first surface comprises one of an aspherical surface, a spherical surface, or a diffractive surface.

5. The ophthalmic lens according to any of claims 1 to 4, wherein the first meridian is a sphere meridian and the second meridian is a cylinder meridian.

6. The ophthalmic lens of claim 1, wherein:

   the first surface is an aspherical front surface;
   the second surface is a rear surface; and

wherein the first meridian is a sphere meridian and the second meridian is a cylinder meridian.

7. The ophthalmic lens according to claim 6, wherein the ophthalmic lens comprises a contact lens.

8. The ophthalmic lens according to claim 7, wherein the contact lens comprises a soft or hybrid contact lens.

9. The ophthalmic lens according to any preceding claim, wherein the ophthalmic lens has a spherical power from -9D to +6D.

10. The ophthalmic lens according to any preceding claim, wherein the ophthalmic lens has a cylindrical power from -0.75D to -3.25D.

11. The ophthalmic lens according to any preceding claim, wherein the ophthalmic lens exhibits about zero spherical aberration along the first meridian.

12. The ophthalmic lens according to any preceding claim, wherein the ophthalmic lens exhibits zero spherical aberration along the first meridian.

13. The ophthalmic lens according to any preceding claim, wherein the ophthalmic lens exhibits less than .001 D/mm^2 spherical aberration along the second meridian.

14. The ophthalmic lens according to any of claims 1 to 12, wherein the ophthalmic lens exhibits less than .05D/mm^2 spherical aberration along the second meridian when configured to exhibit a cylindrical optical power of -3.25D.

15. The ophthalmic lens according to any of claims 1 to 12, wherein the ophthalmic lens exhibits less than .04D/mm^2 spherical aberration along the second meridian when configured to exhibit a cylindrical optical power of between -2.75D and -3.25D.

16. The ophthalmic lens according to any of claims 1 to 12, wherein the ophthalmic lens exhibits less than .002D/mm^2 spherical aberration along the second meridian when configured to exhibit a cylindrical optical power of between -1.75D and -3.25D.

17. The ophthalmic lens according to any of claims 1 to 12, wherein the ophthalmic lens exhibits less than .001 D/mm^2 spherical aberration along the second meridian when configured to exhibit a cylindrical optical power of between -0.75D and -3.25D.

# FIG. 1

Sphere Meridian Curve

Cylinder Meridian Curve

# FIG. 2

Spherical

Symmetry Axis

Aspherical

# FIG. 3

Sphere Curve

Cylinder Curve

Atoric Surface

# FIG. 4

# FIG. 5

# FIG. 6

# FIG. 7A

# FIG. 7B

# FIG. 8

# FIG. 9A

Front
surface

Back
surface

Atoric
surface

# FIG. 9B

Front
surface

Back
surface

Atoric
surface

# FIG. 10A

Legend: —●— -0.75  —◆— -1.75  ----■---- -2.75  ······○······ -3.25

X-axis: Lens spherical power (D)
Y-axis: Lens spherical aberration (D/mm$^2$)

# FIG. 10B

Legend: —●— -0.75  —◆— -1.75  ----■---- -2.75  ······○······ -3.25

X-axis: Lens spherical power (D)
Y-axis: Lens spherical aberration (D/mm$^2$)

# FIG. 11A

# FIG. 11B

Europäisches
Patentamt

European
Patent Office

Office européen
des brevets

# EUROPEAN SEARCH REPORT

Application Number

EP 18 19 1430

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2010/039839 A1 (BAUSCH & LOMB [US]; KINGSTON AMANDA CHRISTINE [US]; ALTMANN GRIFFITH E) 8 April 2010 (2010-04-08)<br>* claims 1,4,5,9; tables 1,2 *<br>* page 6, line 28 - page 7, line 18 *<br>* page 7, two last paragraphs with page 8, first three paragraphs * | 1-17 | INV.<br>G02C7/04<br>A61F2/16 |
| X | WO 2009/131905 A1 (BAUSCH & LOMB [US]; ZUBA JENNIFER [US]; GREEN TIMOTHY [US]) 29 October 2009 (2009-10-29)<br>* page 2, line 21 - line 27; claims 1-8 *<br>* page 5, second paragraph *<br>* paragraph bridging pages 5 and 6 *<br>* page 7, fourth paragraph * | 1-17 | |
| X | WO 2006/064384 A2 (AUGEN OPTICOS S A DE CV [MX]; LANDGRAVE ENRIQUE [MX]; VILLALOBOS ANTON) 22 June 2006 (2006-06-22)<br>* figures 4a,4b,5a,5b; tables 2a, 2b *<br>* abstract * | 1-17 | |
| | | | TECHNICAL FIELDS SEARCHED (IPC) |
| A | FR 1 552 022 A (SOCIETE DES LUNETIERS TEMKINE & CIE) 3 January 1969 (1969-01-03)<br>* the whole document * | 1-17 | G02C<br>A61F |

The present search report has been drawn up for all claims

| Place of search | Date of completion of the search | Examiner |
|---|---|---|
| Munich | 15 January 2019 | Girardin, François |

EPO FORM 1503 03.82 (P04C01)

## ANNEX TO THE EUROPEAN SEARCH REPORT
## ON EUROPEAN PATENT APPLICATION NO.

EP 18 19 1430

This annex lists the patent family members relating to the patent documents cited in the above-mentioned European search report.
The members are as contained in the European Patent Office EDP file on
The European Patent Office is in no way liable for these particulars which are merely given for the purpose of information.

15-01-2019

| Patent document cited in search report | | Publication date | Patent family member(s) | | Publication date |
|---|---|---|---|---|---|
| WO 2010039839 | A1 | 08-04-2010 | CA | 2738901 A1 | 08-04-2010 |
| | | | CN | 102171599 A | 31-08-2011 |
| | | | EP | 2335112 A1 | 22-06-2011 |
| | | | JP | 2012504785 A | 23-02-2012 |
| | | | KR | 20110067142 A | 21-06-2011 |
| | | | US | 2010079723 A1 | 01-04-2010 |
| | | | WO | 2010039839 A1 | 08-04-2010 |
| WO 2009131905 | A1 | 29-10-2009 | US | 2009262301 A1 | 22-10-2009 |
| | | | WO | 2009131905 A1 | 29-10-2009 |
| WO 2006064384 | A2 | 22-06-2006 | EP | 1846800 A2 | 24-10-2007 |
| | | | US | 2006132708 A1 | 22-06-2006 |
| | | | WO | 2006064384 A2 | 22-06-2006 |
| FR 1552022 | A | 03-01-1969 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Normal-eye Zernike coefficients and root-mean-square wavefront errors. *J Cataract Refract Surg.,* December 2006, vol. 32 (12), 2064-74